# EUROPEAN PATENT SPECIFICATION

(11) **EP 0 598 009 B1**
(45) Date of publication and mention of the grant of the patent: **16.10.1996**
(21) Application number: 92917489.4
(22) Date of filing: 03.08.1992
(51) Int. Cl.: C12P 19/62

(54) **CONTINUOUS NATAMYCIN PRODUCTION**
KONTINUIERLICHE NATAMYCINPRODUKTION
PRODUCTION CONTINUE DE NATAMYCINE

(30) Priority: 05.08.1991 US 740536
(43) Date of publication of application: 25.05.1994
(73) Proprietor: BIO-TECHNICAL RESOURCES L.P., Manitowoc, WI 54220 (US)
(72) Inventor: OLSON, Phillip, Terry, Manitowoc, WI 54220 (US)
(74) Representative: Woodcraft, David Charles
(86) International application number: US9206499
(87) International publication number: WO9303171

(56) References cited:
- WO-A-84/00777
- GB-A- 846 933
- US-A- 3 015 612
- US-A- 4 167 450
- DIALOG INFORMATION SERVICES, file 5, BIOSIS, Dialog accession no. 4939650, Biosis accession no. 80066961, C.R. ROBERTSON et al.; Biotechnol Boieng 27(7), 1985, 1012-1020

## Description

The present invention relates to the production of natamycin by continuous fermentation of an inoculum to obtain continuously high yields of natamycin over a prolonged period of time.

Natamycin is a member of the polyene family of antimycotics. The compound natamycin is a tetraene with a molecular weight of about 666, empirical formula corresponding generally to C₃₃H₄₇NO₁₃, and it contains a glycosidically-linked carbohydrate moiety, mycosamine. Natamycin (also called pimaricin) has an isoelectric point of about pH 6.5, and the structure exists typically in two configurations: the enol-structure and the keto-structure.

A conventional batch-type fermentation process for producing natamycin is described in American Cyananfid's British Patent No. 846,933 (1960).

Despite the antibiotic and anti-fungal value of natamycin, very little commercial use has been made of this product. One major reason for the limited use is a prohibitively high manufacturing cost.

It is an object of the present invention to overcome the inefficiencies of conventional processes and provide a continuous process for producing useful quantities of natamycin in a cost-effective manner by propagating and fermenting an inoculum in predetermined media.

EP-A-600 983 and EP-A-597 988 were filed on the same day and by the same applicant as the present application, they both concern aspects of the production of natamycin by fermentation.

### SUMMARY OF THE INVENTION

The present invention relates to the fermentation preparation of natamycin wherein the fermentation is carried out continuously. The fermentation process comprises two basic phases. The first phase comprises culture propagation, whereas the second phase comprises natamycin production. The process results in continuous high yields of natamycin, namely about 4 through at least about 12 g/l over a prolonged fermentation period.

The present invention continuously ferments an organism capable of producing natamycin. Particularly the present invention is directed to preparing (e.g., sporulation) and propagating an inoculum comprising a *Streptomyces* species that, during fermentation, produces natamycin.

A high yield of natamycin is important economically because of the enhanced ease of recovery of natamycin from the fermentation broth. Further, improved cost-effectiveness of the fermentation equipment and more efficient use of the production medium, are achievable by the present continuous production. A continuous fermentation process also decreases the number of inoculum preparations required in comparison to a series of batch operations.

### BRIEF DESCRIPTION OF THE DRAWINGS

Fig. 1 is a schematic block diagram of the process which may be used in the invention for inoculum propagation.

Fig. 2 is a graphical representation of the two phases which occur during the continuous natamycin fermentation process of the invention.

### DETAILED DESCRIPTION OF THE INVENTION

In accordance with the present invention, an organism capable of producing natamycin is placed into contact with a predetermined medium to produce an inoculum, and then into a predetermined fermentation production medium that will support maximum metabolic activity of the organism during further propagation and continuously produce natamycin. During the continuous fermentation, the organism transforms at least a portion of the predetermined production medium into natamycin.

The fermentation process of the invention comprises two phases. The first phase comprises propagation of an inoculum containing a culture of the *Streptomyces* species which may be accompanied by some natamycin production. The natamycin production rate during the first phase increases from zero to the steady state rate of the second phase. The second phase is primarily directed to an equilibrium or steady state natamycin production at a high rate which achieves a fermentation broth concentration of about 4 through at least about 12 g/l. These two phases may overlap with some natamycin production taking place when culture propagation is the major activity, and culture growth continuing throughout the natamycin production.

During the second phase, a predetermined equilibrium is reached and maintained so that culture propagation and high natamycin production rates remain approximately constant. This culture propagation/high natamycin production rate equilibrium is achieved by 1) continuously adding to the fermenter, starting at the appropriate time, the continuous production medium to maintain a constant rate of culture propagation and natamycin production and 2) removing the natamycin containing fermentation broth. In the continuous or second phase, broth concentrations and volume are maintained constant However, if desired, a different natamycin production rate may be achieved by either modifying the composition or rate of medium addition.

Theoretically, the equilibrium during continuous production phase can continue indefinitely. However, in practice of the invention, the continuous phase is limited by contamination or equipment malfunctions, and is normally continued for at least about 40 days or until natamycin production decreases to an uneconomical yield rate.

The key aspects of the invention which permit continuous fermentation are characterized by:
1. Composition of the inoculum medium which is used to propagate the culture that is introduced into the fermentor to begin the first phase of fermentation.
2. At least 2 phases of fermentation; a first phase which comprises a non-steady state culture growth/natamycin production during which the natamycin production rate increases and a second phase comprising culture propagation/high natamycin production at a predetermined equilibrium.
3. Composition of the fermentation media.
4. Constant fermentation broth volume, composition and culture concentration during the second phase which is achieved by addition of fermentation medium balanced by an equal volume removal of fermentation broth.
5. Medium addition/broth removal rates selected to maximize economical medium consumption and natamycin production.
6. If desired, the natamycin may be recovered or separated from the removed fermentation broth.

Any organism which comprises a natamycin producing *Streptomyces* species can be used in accordance with the invention. A preferred *Streptomyces* species comprises *Streptomyces gilvosporeus* which has been deposited previously with the American Type Culture Collection (ATCC) in Rockville, Maryland, United States of America, that is registered as ATCC No. 13326.

An inoculum to be fermented is prepared from a spore suspension of the appropriate spores. The inoculum of the appropriate *Streptomyces* species is subsequently fermented which produces high yields of natamycin when placed into the continuous fermentation medium of the invention. The inoculum is typically exposed to a series of propagation steps wherein each step increases the quantity of the natamycin producing *Streptomyces* cells. After the quantity of *Streptomyces* cells is adequate, the *Streptomyces* is exposed to an environment and/or a medium which is designed to achieve continuous natamycin production when the *Streptomyces* species ferments.

### Spore Suspension

The inoculum is started by collecting the spores of a natamycin producing *Streptomyces* species which was obtained from the American Type Culture Collection. The spores are germinated to produce an actively growing culture of the *Streptomyces* species. A sterilized (e.g., autoclaved), agar slant is inoculated heavily with the actively growing culture of the *Streptomyces* species (e.g., *Streptomyces gilvosporeus* or any other natamycin producing species), and incubated until the slant is covered substantially entirely with spores. The spores on the agar slant are scraped into a small amount of a liquid, such as water (e.g., distilled water), nutrient medium, etc., to produce an aqueous spore suspension. The resulting spore suspension is propagated to produce the inoculum for the fermentation operation (i.e., natamycin production). For achieving the best results, the spore suspension used to begin inoculum propagation should contain a spore concentration of about 10⁵-10¹⁰ CFU/ml, and, normally, at least about 10⁸ CFU/ml.

A number of agar slant media can be used to promote sporulation of the culture of the *Streptomyces* species (e.g., S. *gilvosporeus),* which will be used to form the spore suspension Appropriate agar slant mediums typically comprise at least one member of the following group: yeast malt agar, Hickey-Turner agar, GYA agar, Pridham agar, potato dextrose agar, Bennett's agar, etc.

A high concentration (e.g., 10⁸ CFU/ml), of viable spores within the spore suspension is a key aspect of the present invention. First, if the concentration of spores is too low, it takes much longer to obtain, through inoculum propagation, the quantity of *Streptomyces* cells sufficient for cost-effective natamycin fermentation production. Second, a reduced quantity of spores within the suspension lengthens the total inoculum propagation time and increases the likelihood of a contamination (e.g., by an unwanted organism). Further, a low spore concentration within the suspension may tend to promote the formation of large, tightly packed mycelial pellets. These pellets are unsuitable for obtaining high yields of natamycin due to problems associated with oxygen transfer, mass transfer of nutrients into the pellets, etc. Should the size of mycelial pellets become undesirable, the pellets can be broken apart physically, such as by using a shear force (e.g., blending).

### Inoculum Propagation

The aqueous spore suspension (e.g., S. *gilvosporeus),* discussed above, is germinated and cell multiplication continued until the number of organisms is adequate to be used as an aqueous inoculum for fermentation production of natamycin. A suitable inoculum cell density comprises a dry cell weight of about 1-5 g/l and is used at a volume of 0.1-10% of the natamycin production medium volume.

The aqueous medium used for inoculum propagation determines the cell density and the metabolic state of the inoculum (e.g., an adequate density of healthy cells is desirable). A sufficient amount of protein nitrogen which contains complex growth factors (e.g., vitamins), inorganic elements (e.g., potassium, sodium, calcium, etc.), and trace elements (e.g., boron, cobalt, iron, copper, zinc, etc.), that are commonly present in the protein nitrogen source are needed to achieve an inoculum possessing the desired cell density and metabolic state. The protein nitrogen source may be any source that will propagate the spore suspension into an inoculum that will ferment to produce the desired high yields of natamycin.

A metabolizable source of carbon must also be supplied to the aqueous inoculum medium in an amount which is sufficient to achieve the desired inoculum cell density. For best results, the carbon source should not be depleted completely during the inoculum propagation. Depletion of the carbon source tends to alter adversely the metabolic state of the inoculum, which may lead to reduced yields of natamycin during fermentation.

Although a variety of aqueous inoculum media can be used effectively in accordance with the present invention, to obtain high yields of natamycin it is advantageous to use predetermined amounts of medium ingredients.

A suitable medium for inoculum propagation may be prepared in water (e.g., low mineral content water, distilled water, etc.), and comprises:
a) a protein nitrogen source in an amount from about 2-16 g/l, normally about 8 g/l; and
b) a metabolizable carbon source present in an amount which is sufficient to avoid total carbon depletion, usually 5-30 g/l of medium, and normally about 15 g/l.

Two specific compositions of a medium appropriate for inoculum propagation are given below.

| Composition 1 | Quantity |
|---|---|
| Difco® "Bacto®" peptone | 5 g/l |
| Corn steep liquor | 3 g/l |
| Sodium chloride | 10 g/l |
| Glucose | 15 g/l |

| Composition 2 | Quantity |
|---|---|
| Hormel peptone PSR 5 | 8 g/l |
| Sodium chloride | 10 g/l |
| Glucose | 15 g/l |

The inoculum medium which provides nutrients that enhances the production rate of *Streptomyces* cells may be prepared by conventional techniques (e.g., separate or simultaneous sterilization of the carbon and nitrogen sources at temperatures of about 120-140°C). The inoculum medium after sterilization, desirably has a pH of about 7. The spore suspension is introduced to the inoculum medium and the inoculum medium is heated to a temperature of about 25 - 40°C and normally, about 28 - 35°C.

In order to achieve the large volumes of aqueous inoculum which are desirable for the continuous fermentation production of natamycin, several inoculum propagation steps are required, each carried out in a volume greater than the previous step. For example, the inoculum propagation may be conducted in a manner which achieves an exponential increase in the quantity of cells. Particularly, it is advantageous to keep the culture in an exponential growth mode during propagation by effectively increasing the volume of the inoculum during each step of the propagation. This can be done by either minimizing the duration of each step or by This can be done by either minimizing the duration of each step or by minimizing the number of steps. For example, once a predetermined cell density of inoculum has been achieved, the inoculum is transferred to a larger environment (e.g., vessel), for further propagation. By effectively controlling the inoculum propagation a minimum of time and expense is devoted to inoculum propagation and, accordingly, cost-effective natamycin yields during fermentation are increased.

The length of time an individual step in the series of inoculum propagation steps is permitted to continue depends upon the composition of the medium, quantity of *Streptomyces* cells desired, temperature, etc. Typically, an individual propagation step is conducted for about 6 through about at least about 24 hours.

The inoculum propagation process requires aeration of the inoculum. For example, the vessel or flask may be agitated on a rotary shaker at a rate of about 200 rpm. In one aspect of the invention, the inoculum may be agitated by an impeller which is located within the vessel that houses the inoculum while sterile air is forced into the bottom of the vessel.

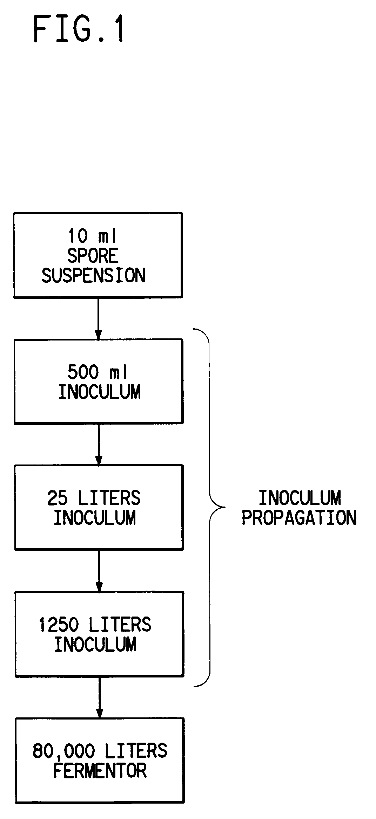

Now referring to Figure 1, this figure is a schematic of the process which may be used to produce the inoculum that is fermented to produce natamycin. Figure 1 illustrates the volumetric increases in the inoculum which are achieved by propagation that are necessary to obtain a quantity of inoculum that is adequate to produce natamycin in a cost-effective manner. For example, the volume of inoculum is increased from 25 liters to 1250 liters by adding the 25 liters of inoculum to a vessel containing 1225 liters of an aqueous inoculum medium.

The propagated inoculum is introduced into a fermentor housing a fermentation medium. Rapid culture propagation continues during the first phase of fermentation, along with increasing natamycin production. The natamycin production rate during the second phase of fermentation, will stabilize usually in about 1 day at a high yield which will continue for several days. The natamycin concentration in the broth increases at approximately a constant rate until the natamycin production rate starts to decrease usually because of depletion of one or more of the inoculum medium ingredients.

### Continuous Natamycin Production

The second phase of fermentation begins before or shortly after the rate of natamycin production begins to decline. The natamycin production rate is determined by continuously monitoring the natamycin concentration in the fermentation broth. When the second phase has been reached, the rate of medium addition and broth removal are coordinated. By monitoring the removed fermentation broth composition, the medium addition/ broth removal rates may be adjusted to obtain optimum usage of the fermentation medium. Maximum possible medium utilization is achieved when any ingredient that is being added in the medium decreases to zero steady state concentration in the fermentation broth. Depending on medium and equipment costs, it may be economically desirable to add medium and remove broth at a rate somewhat faster than that which will give maximum possible medium utilization.

The medium addition and broth removal may be performed by any acceptable technique. For example, the medium addition and/or broth removal may be accomplished by using a pump (e.g., a vacuum pump), pressure, gravity, etc.

If desired, second phase fermentation medium can be adjusted or even significantly changed in composition or feed rate at any time to optimize production economics of the continuous production. Also, depending primarily on the equipment being used, it may be desirable to add medium and/or remove broth intermittently as long as the overall average broth volume remains approximately constant. Furthermore, the second phase medium addition can be started early, toward the end of the first phase; but the second phase does not commence until the equilibrium or steady state discussed above is reached.

During the second phase, fermentation broth containing natamycin product, biomass (e.g., *Streptomyces* cells), and production medium is continuously removed, and natamycin product is recovered (e.g., filtered, centrifuged, etc.) from the removed broth either mixed with the biomass and/or extracted as natamycin (e.g., in a crystalline morphology). The natamycin may be recovered by any suitable technique which obtains natamycin in a useful form and quantity. Should the natamycin be mixed with the biomass, if desired, the natamycin may be recovered from the biomass by any suitable technique.

The continuous steady state second fermentation phase may be sustained for a prolonged period of time, primarily being limited only by the introduction of contaminants or equipment malfunction (e.g., the present invention may continue for at least about forty days).

Continuous natamycin production is conducted in a fermentation vessel which is capable of housing the fermentation process. One element important for achieving maximum yields of natamycin is the composition of the aqueous fermentation medium. The fermentation medium must contain the proper amounts of metabolizable carbon and protein nitrogen. Also, it is desirable that the medium contains complex growth factors (e.g., vitamins), and inorganic elements (e.g., potassium, sodium, calcium, etc.), and trace elements (e.g., boron, cobalt, iron, copper, zinc, etc.), that are commonly present in the protein nitrogen source.

A suitable medium for fermentation may be prepared in water (e.g., low mineral content tap water, distilled water, etc.), and comprises:
(a) about 80-250 g/l of a metabolizable carbon source; and
(b) at least 15 g/l and, normally about 20 g/l through 80 g/l, of a protein and trace ingredients. The protein nitrogen source may comprise a non-yeast protein nitrogen component and a yeast protein nitrogen component. These two protein nitrogen components are usually present in the ratio ranging, respectively, from about 5:1 to 11:1 based on protein contents, and for best results generally about 8:1

These metabolizable carbon source and protein nitrogen source quantities are continuously maintained in the fermentation medium throughout the second phase by continuous and coordinated addition of the appropriate source(s). The quantity of a particular source in the fermentation medium can be determined by monitoring the broth being removed and making any appropriate adjustment.

The protein nitrogen source may be continuously supplied from a wide range of sources. For example, soy protein products (e.g., isolates, flours, meals, etc.), may comprise the non-yeast protein nitrogen source (e.g., desirable natamycin yields are obtained with a soy protein source comprising 80-95% protein). The protein nitrogen may also comprise beef extract, protein hydrolysates (e.g., peptones), and/or yeast (e.g., extracts, autolysates, etc.).

As discussed above, the production medium must also include a source of carbon which is metabolizable by the *Streptomyces species.* The carbon source may be supplied in any expedient form such as glucose, polysaccharide, corn and potato starches, etc.

Moreover, in one aspect of the invention, it is not necessary to initially introduce the entire quantity of the carbon source which is required to produce natamycin, as a starting component of the natamycin production medium (e.g., the initial quantity of the carbon source is not adequate for completion of the fermentation). In this aspect of the present invention, during phase 1, carbon source addition may be performed during the natamycin production so as to maintain a quantity of carbon source of about 5-40 g/l, and usually 20 g/l. Thus, an appropriate quantity of a suitable carbon source is added to the fermentation medium initially and again after the fermentation has begun. During phase 2, continuous natamycin production, the carbon source is added at a predetermined rate which is proportional to the rate of addition of the natamycin production medium. Preferably, the rate of carbon source addition is sufficient to provide a quantity of carbon source to maintain a continuous high rate of natamycin production without unconsumed carbon source being removed from the fermentor (e.g., the quantity of the carbon source substantially equates to the particular quantity of carbon source within the fermentation medium which is necessary to sustain the fermentation process).

In another aspect of the invention, the carbon source can be added as part of the production medium, in which case the rate of carbon source addition is determined by the rate of addition of the production medium.

The natamycin production medium, which provides nutrients for the *Streptomyces* fermentation and natamycin production is prepared by conventional techniques (e.g., separate or simultaneous sterilization of the carbon and nitrogen sources at temperatures of about 120-140°C). The production medium, after sterilization, desirably has a pH of about 7. In one aspect of the invention, the fermentation medium may be sterilized en route to the fermentor. For example, the fermentation medium, which is being fed continuously to the fermentor, may be passed through a pipe, conduit or other suitable means which is capable of sterilizing the fermentation medium (e.g., a continuous sterilizer).

The inoculum is introduced into a fermentation vessel until a concentration of about to 0.1-10%, usually about 2%, by volume is achieved in the production medium (e.g., the quantity of inoculum may be sufficient to inoculate a plurality of continuous fermentors). The remainder of the volume of the fermentor comprises the fermentation medium Any technique is acceptable for introducing the inoculum to the production medium within the fermentor which delivers the inoculum in an active metabolic state.

The fermentation or production medium is brought to a temperature of about 25°-40°C, and normally 28°-35°C.

Oxygen is supplied to the natamycin production medium during fermentation. It is advantageous to maintain a dissolved oxygen level in the production medium of about 20%-80% of air saturation during the major portion of the fermentation. The ability to achieve a suitable dissolved oxygen level may be enhanced by proper coordination of the aeration and/or agitation rate. For example, the fermentation or production medium is aerated by forcing air (e.g., sterile air), through the fermentation medium, usually at a rate of about 03 through at least about 1.0 volumes of air per volume of fermentation medium. In one aspect of the invention it is desirable to agitate the fermentation medium while being aerated. Further, the rate of aeration may also be sufficient to cause agitation of the fermentation medium.

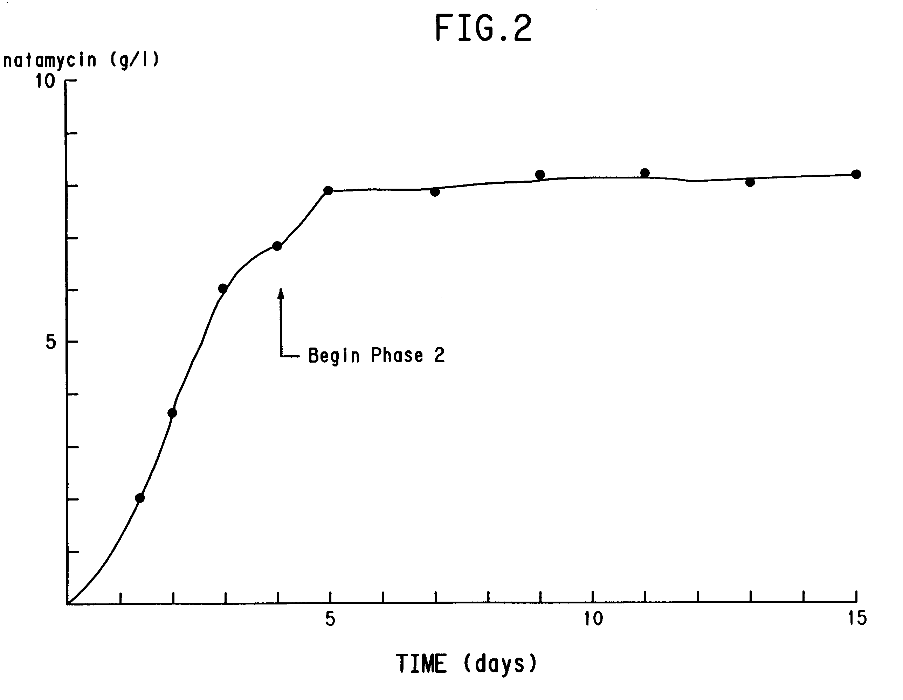

Referring now to Fig. 2, the relationship between time and natamycin production rate is shown for each of the two basic phases of the invention. The first phase includes propagation of the *Streptomyces* species which is accompanied by some natamycin production. The second phase, continuous production of natamycin, is established when an equilibrium is achieved between the medium addition and the broth removal. The second phase is continued until natamycin is no longer produced in a cost effective manner (e.g., a contamination renders the process inefficient).

In one aspect of the invention, it may be desirable to add an anti-foaming agent (e.g., a silicone de-foamer), to the fermentation medium in an amount of about 0.01%-1% by volume of the fermentation or natamycin production medium when it is desirable to control foaming.

The natamycin production medium after inoculum addition has a pH of about 7.0, which typically decreases slowly during fermentation to about 4.5. The lowered pH is a result of the metabolic activities of the *Streptomyces* species. Depending upon the end-use of the fermentation broth, it may be desirable for the fermentation medium to have a lowered pH. However, the pH may be increased or controlled if desirable by the timely addition of a basic substance (e.g., potassium hydroxide may be added continuously or intermittently to the fermentation medium to maintain a certain pH). Also, the pH can be increased or decreased by altering the medium composition and/or the rate of medium addition/broth removal.

When the present invention is practiced appropriately (e.g., effective handling of the *Streptomyces* inoculum, coordinated addition of media, and broth removal, etc.), the resultant fermentation broth will normally include at least about 4 g/l of natamycin. In certain cases, the level of natamycin production may range from about 7 g/l through at least about 12 g/l.

The invention is demonstrated by the following Example which is intended to illustrate, not limit, the scope of the contemplated equivalents. Unless specified otherwise, commercially available reagent grade materials were used to conduct the following Examples.

### EXAMPLE 1

In the following Example, agar slants of the following general composition are prepared in distilled water. Sterilization is at about 121°C for about 15 minutes.
3 g/l yeast extract (Difco® "Bacto®" Yeast Extract)
3 g/l malt extract (Difco Malt Extract)
5 g/l peptone (Difco® "Bacto®" peptone)
10 g/l glucose
15 g/l Agar

An inoculum medium of the following general composition were prepared in distilled water, and the pH was adjusted to about 7.0 with potassium hydroxide. Sterilization is at about 121°C for about 15 minutes.
15 g/l glucose
10 g/l sodium chloride
6 g/l corn steep liquor (PPM, corn Steep Liquid)
5 g/l peptone (Difco® "Bacto®" peptone)

*Streptomyces gilvosporeus,* ATCC Reg. No. 13326, obtained from ATCC as a freeze-dried spore suspension was used as the culture source. The culture was grown in the inoculum medium and held on the agar slants at about 25°C for sporulation. The development of the inoculum started with the agar slant cultures.

Agar slants for this production run inoculum sporulated heavily within about 10 days. Spores were scraped off these agar slants into inoculum medium in order to achieve a spore suspension concentration of about 108 CFU/ml. Approximately 2 ml of the spore suspension was added to about 100 ml of inoculum medium in a 500 ml baffled flask. This culture was incubated in three steps for a total incubation of about 48 hours at about 29°C and agitated at about 240 rpm on a rotary shaker. The first two steps are 12 hours each, and the third step is about 24 hours. For the second and third steps, fresh inoculum medium was introduced with about 2% volume from the proceeding step. The final inoculum culture thus produced was used to inoculate the production medium at the required inoculum concentration.

The first phase natamycin production medium was of the following general initial composition:
195 g/l soy protein isolate (ADM, "Profam®" S970)
4.5 g/l yeast extract (Stauffer, Type KAT)
02 m/l defoamer (Mazu, DF 289)

Approximately 600 ml of medium was prepared in distilled water in a 1 liter fermenter and the pH was adjusted to about 7.6 with potassium hydroxide. The 1-liter fermenter was then sterilized for about 15 minutes at about 121°C. Glucose, which also is part of the first phase medium, was sterilized separately as an approximately 50% solution in distilled water, and was added to the initial medium at a concentration of about 40 g/l.

Before inoculation, the production medium was heated to about 29°C, the aeration rate was set at about 250 ml/min, and the agitation rate was about 500 rpm.

Inoculum comprising *Streptomyces gilvosporeus,* ATCC Reg. No. 13326, about 2% by volume of the production medium, was then added to the fermentation vessel to start phase 1 of the production cycle. Glucose was added beginning at about 40 hours after inoculation in order to maintain a concentration in the first fermentation phase of about 20 g/l glucose. This was done by feeding glucose to the fermentation vessel at a rate of about 1 g/l-hr.

Approximately 72 hours after inoculation at the start of the first phase of fermentation, the natamycin production rate began to decline. The concentration of the broth was about 3.9 g/l. Second phase medium was added and fermentation broth was withdrawn from the fermenter at the rate of about 12 ml/hr to maintain a constant broth volume of about 600 ml. The production medium comprised approximately 19.5 g/l soy protein isolate, 4.5 g/l yeast extract, 50 g/l glucose and 0.2 ml/l of defoamer.

The additions and withdrawal were continued at about 12 ml/hour, and the fermentation continued until about 200 hours after the start of the first fermentation phase. Natamycin production was only mediocore during this period, the concentration of natamycin in the broth being about 4.5 g/l. This product was recovered from the withdrawn broth.

To increase the rate of natamycin production, the medium was changed to the following general composition: 26 g/l soy protein isolate, 6 g/l yeast extract, 80 g/l glucose and 03 ml/l of defoamer. Additions of the increased nutrient medium and withdrawal of broth were continued at about the same rate. At about 250 hours from the start of phase one, the natamycin in the withdrawn broth had increased in concentration to the high yield of about 8.5 g/l, which concentration remained approximately constant throughout the remainder of fermentation. At about 450 hours, the process was discontinued.

### EXAMPLE 2

The process substantially, in accordance with Example 1, was performed through phase 1 with the exception that the phase 1 production medium comprised approximately: 26.0 g/l soy protein isolate, 6.0 g/l yeast extract and 03 ml/l defoamer.

After about 70 hours from the start of phase 1, the natamycin concentration reached about 6.0 g/l and phase 2, continuous operation was begun. The medium for phase 2 comprised approximately: 26.0 g/l soy protein isolate, 6.0 g/l yeast extract, 80 g/l glucose and 03 ml/l defoamer. The medium was added at a rate of about 12 ml/hr and fermentation broth was withdrawn from the fermenter to maintain a constant volume of about 600 ml. At about 117 hours a steady state equilibrium had been achieved and the natamycin concentration was about 8.0 g/l. This remained constant until about 250 hours, at which time the fermentation was stopped.

## Claims

1. A process for preparing natamycin by fermentation comprising:
(a) at least 2 phases of fermentation, the first phase comprising a non-steady state culture growth and natamycin production phase during which natamycin production increases the concentration in the fermentation broth; and a second phase comprising steady state culture propagation and natamycin production rate, wherein during the second phase fermentation broth composition and volume remain substantially constant;
(b) utilizing an inoculum comprising a natamycin producing *Streptomyces* strain in an inoculum medium comprising 2 to 16 g/l of a protein nitrogen source and a metabolizable carbon source in adequate quantity to avoid total carbon depletion during inoculum preparation, said inoculum being added to the first phase fermentation medium in an amount of 0.1 to 10% by volume;
(c) utilizing in said first fermentation phase a fermentation medium comprising at least 15 g/l of protein nitrogen and a metabolizable carbon source sufficient to maintain a carbon source concentration in the fermentation broth in the range of 5 to 40 g/l;
(d) continuously adding to the first phase fermentation broth a second phase fermentation medium capable of supporting high rate natamycin production, said medium comprising at least 15 g/l of protein nitrogen source and a metabolizable carbon source in an amount to sustain the natamycin production;
(e) during addition of said second phase medium steady state conditions of fermentation broth composition and volume are achieved by equal volume additions of medium and removals of fermentation broth; and
(f) recovering natamycin from the removed fermentation broth.

2. The process of claim 1 further comprising aerating the fermentation medium at a rate sufficient to cause the fermentation medium to be agitated.

3. The process of claim 1 or 2 further comprising sterilizing the fermentation medium en route.

4. The process of any one of the preceding claims wherein the protein nitrogen source comprises a non-yeast nitrogen component and a yeast nitrogen component.

5. The process of claim 4 wherein the two protein nitrogen components are present in the ratio of non-yeast nitrogen component to yeast nitrogen component of from 5:1 to 11:1.

6. The process of any one of the preceding claims wherein the fermentation is conducted to achieve a concentration of natamycin in the fermentation broth of at least 4 g/l.

7. The process of claim 6 wherein the concentration of natamycin is at least 7 g/l.

8. The process of any one of the preceding claims wherein the natamycin producing *Streptomyces* species is *Streptomyces gilvosporeus*.

## Patentansprüche

1. Verfahren zur Herstellung von Natamycin durch Fermentation, welches umfaßt:
(a) wenigstens 2 Fermentationsphasen, wobei die erste Phase ein Nichtgleichgewichtszustand-Kulturwachstum und eine Natamycin-Produktionsphase, während der die Natamycin-Produktion die Konzentration in der Fermentationsbrühe vergrößert, umfaßt, und eine zweite Phase, die eine Gleichgewichtszustand-Kulturvermehrung und eine Natamycin-Produktionsgeschwindigkeit umfaßt, bei der während der zweiten Phase die Fermentationsbrühenzusammensetzung und das Volumen im wesentlichen konstant bleiben;
(b) Verwendung eines Inoculums, welches einen Natamycinproduzierenden *Streptomyces-*Stamm in einem Inoculummedium umfaßt, welches 2 bis 16 g/l einer Proteinstickstoffquelle und eine metabolisierbare Kohlenstoffquelle in entsprechender Menge umfaßt, um eine völlige Kohlenstoff-Verarmung während der Inoculumherstellung zu vermeiden, wobei das Inoculum dem Erstphasen-Fermentationsmedium in einer Menge von 0,1 bis 10 Vol.-% zugesetzt wird;
(c) Verwendung in der ersten Fermentationsphase eines Fermentationsmediums, welches wenigstens 15 g/l Proteinstickstoff und eine metabolisierbare Kohlenstoffquelle umfaßt, die ausreicht, um eine Kohlenstoffquellenkonzentration in der Fermentationsbrühe im Bereich von 5 bis 40 g/l aufrechtzuerhalten;
(d) kontinuierliche Zugabe zu der Erstphasen-Fermentationsbrühe eines Zweitphasen-Fermentationsmediums, das in der Lage ist, hohe Natamycin-Produktionsgeschwindigkeiten zu unterstützen, wobei das Medium wenigstens 15 g/l Proteinstickstoffquelle und eine metabolisierbare Kohlenstoffquelle in einer Menge umfaßt, um die Natamycin-Produktion zu unterhalten;
(e) während der Zugabe des Zweitphasenmediums werden die Bedingungen des Gleichgewichtszustandes der Fermentationsbrühenzusammensetzung und des Volumens durch volumengleiche Mediumzugaben und Fermentationsbrühenentnahmen erreicht; und
(f) Gewinnen von Natamycin aus der entnommenen Fermentationsbrühe.

2. Verfahren nach Anspruch 1, das außerdem das Belüften des Fermentationsmediums mit einer Geschwindigkeit, die ausreicht, um ein Rühren des Fermentationsmediums zu bewirken, umfaßt.

3. Verfahren nach Anspruch 1 oder 2, das außerdem das Sterilisieren des Fermentationsmediums während des Verfahrens umfaßt.

4. Verfahren nach einem der vorhergehenden Ansprüche, bei dem die Proteinstickstoffquelle eine Nichthefe-Stickstoffkomponente und eine Hefe-Stickstoffkomponente umfaßt.

5. Verfahren nach Anspruch 4, bei dem die beiden Proteinstickstoffkomponenten in dem Verhältnis von Nichthefe-Stickstoffkomponente zu Hefe-Stickstoffkomponente von 5:1 -bis 11:1 vorhanden sind.

6. Verfahren nach einem der vorhergehenden Ansprüche, bei dem die Fermentation durchgeführt wird, um eine Natamycin-Konzentration in der Fermentationsbrühe von wenigstens 4 g/l zu erzielen.

7. Verfahren nach Anspruch 6, bei dem die Natamycin-Konzentration wenigstens 7 g/l beträgt.

8. Verfahren nach einem der vorhergehenden Ansprüche, bei dem die Natamycin-produzierende *Streptomyces*-Spezies *Streptomyces gilvosporeus* ist.

## Revendications

1. Procédé de préparation de natamycine par fermentation comprenant :
(a) au moins deux phases de fermentation, la première phase comprenant une phase de croissance de la culture et de production de natamycine non stationnaire, au cours de laquelle la production de natamycine augmente la concentration dans le bouillon de fermentation ; et une seconde phase comprenant une propagation de la culture et un taux de production de natamycine à l'équilibre, dans lequel, au cours de la seconde phase, la composition et le volume du bouillon de fermentation restent constants ;
(b) l'utilisation d'un inoculum comprenant une souche de Streptomvces productrice de natamycine dans un milieu d'inoculum comprenant 2 à 16 g/l d'une source d'azote protéique et une source de carbone métabolisable en une quantité permettant d'éviter l'épuisement total du carbone pendant la préparation de l'inoculum, ledit inoculum étant ajouté au milieu de fermentation de la première phase en une quantité comprise entre 0,1 et 10 % en volume ;
(c) l'utilisation dans ladite première phase de fermentation d'un milieu de fermentation comprenant au moins 15 g/l d'azote protéique et une source de carbone métabolisable suffisante pour maintenir une concentration de source de carbone dans le bouillon de fermentation comprise entre 5 et 40 g/l ;
(d) l'addition continuelle au bouillon de fermentation de la première phase d'un second milieu de fermentation de seconde phase capable de maintenir un taux élevé de production de natamycine, ledit milieu contenant au moins 15 g/l de source d'azote protéique et une source de carbone métabolisable en une quantité permettant d'entretenir la production de natamycine ;
(e) au cours de l'addition dudit milieu de seconde phase, les conditions d'équilibre de composition et de volume du bouillon de fermentation sont obtenues par des additions de volumes de milieu et de volumes de prélèvement de bouillon de fermentation égaux ; et
(f) la récupération de la natamycine dans le bouillon de fermentation prélevé.

2. Procédé de la revendication 1, comprenant en outre l'aération du milieu de fermentation à un taux suffisant pour entraîner l'agitation du milieu de fermentation.

3. Procédé de la revendication 1 ou de la revendication 2, comprenant en outre la stérilisation du milieu de fermentation sur son trajet.

4. Procédé de l'une quelconque des revendications précédentes, dans lequel la source d'azote protéique comprend un composant d'azote ne provenant pas de levure et un composant d'azote provenant de levure.

5. Procédé de la revendication 4, dans lequel les deux composants d'azote protéique sont présents dans le rapport de composant d'azote ne provenant pas de levure au composant d'azote provenant de levure compris entre 5/1 et 11/1.

6. Procédé de l'une quelconque des revendications précédentes, dans lequel la fermentation est effectuée de façon à obtenir une concentration de natamycine dans le bouillon de fermentation d'au moins 4 g/l.

7. Procédé de la revendication 6, dans lequel la concentration de natamycine est d'au moins 7 g/l.

8. Procédé de l'une quelconque des revendication précédentes dans lequel l'espèce de Streptomyces produisant de la natamycine est Streptomyces gilvosporeus.
